# EUROPEAN PATENT APPLICATION

(11) **EP 3 399 316 A1**
(43) Date of publication of application: **07.11.2018**
(21) Application number: 18171445.2
(22) Date of filing: 08.08.2012
(51) Int. Cl.: G01N 33/68, C07K 16/18, A61K 39/395, A61P 35/04, A61P 9/10, A61P 7/10, A61P 17/02, C07K 16/22, C07K 16/28

(54) **ANGIOPOIETIN-LIKE 4 AND ITS USE IN MODULATING CELL LEAKINESS**

(30) Priority: 08.08.2011 US 201161521031 P
(62) Divisional of application: 12821676.9
(71) Applicant: Nanyang Technological University, Singapore 639798 (SG)
(72) Inventor: Tan, Nguan Soon, Singapore 639798 (SG)
(74) Representative: Winkler, Andreas Fritz Ernst

(57) **Abstract**

Vascular disruption induced by interactions between tumor-secreted permeability factors and adhesive proteins on endothelial cells facilitates metastasis. The role of tumor secreted angiopoietin-like 4 (cANGPTL4) in vascular leakiness and metastasis is controversial due to the lack of understanding of how cANGPTL4 modulates vascular integrity. Here, we show that cANGPTL4 instigated the disruption of endothelial continuity by directly interacting with three novel binding partners, integrin α5β1, VEcadherin and claudin-5, in a temporally sequential manner, thus facilitating metastasis. We showed that cANGPTL4 binds and activates integrin α5β1-mediated Rac1/PAK signaling to weaken cell-cell contacts. cANGPTL4 subsequently associated with and declustered VE-cadherin and claudin-5, leading to endothelial disruption. Interfering with the formation of these cANGPTL4 complexes delayed vascular disruption. *In vivo* vascular permeability and metastatic assays performed using *ANGPTL4*-knockout and wild-type mice injected with either control or ANGPTL4-knockdown tumors confirmed that cANGPTL4 induced vascular leakiness and facilitated lung metastasis in mice. Thus, our findings elucidate how cANGPTL4 induces endothelial disruption. Our findings have direct implications for targeting cANGPTL4 to treat cancer and other vascular pathologies.

## Description

### Cross-reference to related application

This application claims benefit of, and priority from, U.S. provisional patent application No. 61/521,031, filed on 8 August 2011, the contents of which are hereby incorporated herein by reference

### Field

The present invention relates to modulation of cell permeability.

### Background

Endothelial cell leakiness is a phenomenon commonly observed in tumor blood vessels and involved in pathogenesis of asthma, sepsis and cardiovascular disease. Normally, endothelial cells form a tight monolayer that only allows small molecules like ions and solutes to pass through. In pathological cases, increased permeability of the endothelial cells or disruption of the cell-cell junctions caused endothelial leakiness, allowing macromolecules or cells (such as tumor cells and macrophages) to pass through the blood vessel more easily. On the other hand, a leaky endothelial cell layer facilitates drug delivery into target tissues. Therefore, depending of the context and purpose, it may be advantages to either induce or inhibit vascular leakiness.

The endothelium of a blood vessel is made up of adjacent endothelial cells that are tightly packed together. These adjacent cells form adhesive structures, including adherens and tight junctions, which are required to establish cell-polarity, survival as well as to maintain barrier functions. Paracellular and transcellular permeability, which control the movement of molecules in between two cells or across a single cell respectively, regulate the endothelial barrier functions. However in cancer metastasis, paracellular permeability governed by intercellular adherens and tight junctions are always compromised.

Angiopoietin-like 4 (ANGPTL4) is an adipocytokine and also functions as a matricellular protein. ANGPTL4 is identified as prominent gene in a compact *in vivo* hypoxia signature that predicts a poor outcome in multiple tumor types. ANGPTL4 mRNA has been found to be expressed upregulated in the perinecrotic areas of many human tumors, clear-cell renal, oral tongue squamous cell carcinoma and human gastric cancers.

Diabetic ulcers are characterized by an accumulation of devitalized tissue, increased/prolonged inflammation, poor wound-related angiogenesis and reduced matrix deposition. Studies have investigated these alterations to better understand the wound healing abnormalities, and to target therapy specifically aimed at correcting these deficiencies. Recombinant PDGF-BB (becaplermin) is the only growth factor to date approved by the US Food and Drug Administration for the treatment of diabetic foot ulcers. PDGF-B is known to be a potent mitogen and chemotatic agent for stromal cells and may act to increase the wound vascularization by stimulating angiogenesis. There is a need for an alternative treatment or method to accelerate wound repair in diabetic patients.

Tumor metastasis is the main cause of mortality in cancer patients (1). It is determined largely by vasculature leakiness and the critical steps of intravasation and extravasation, which involve the directional migration of tumor cells across the disrupted endothelium. The endothelium provides a semi-permeable boundary between the bloodstream and tumor. The paracellular permeability of the endothelium is mediated primarily by transmembrane endothelial adherens junction (AJ) and tight junction (TJ) proteins that are linked to the actin cytoskeleton (2), and connect adjacent endothelial cells (ECs) (3). Thus, the disruption of major components of AJs and TJs, such as the intercellular VE-cadherin and claudin-5 clusters leads to changes in the actin cytoskeleton, cell shape and the activation of intracellular signaling pathways, which partly via β-catenin initiate gene transcription (4). Apart from AJs and TJs, integrins also strengthen EC-EC contacts and provide a scaffold for cytoskeletal reorganization during cell organization, proliferation and migration (5). Specifically, the expression of integrin α5β1 regulates endothelial monolayer integrity (6). Inherently, metastasis requires communication between tumor cells and ECs that culminates in the disruption of EC-EC contacts and the degradation of the vascular basement membrane. However, the molecular mechanisms that facilitate such communication are poorly understood.

In the tumor microenvironment, interactions among tumor cells, their secreted factors and the endothelium induce vascular permeability to aid tumor metastasis (5, 7). These factors bind to cognate cellular receptors, setting off a cascade of downstream molecular signaling events that determine the outcome of malignancy (8,9). Angiopoietin-like 4 (ANGPTL4) protein has been implicated in cancer (10) however, its precise role in cancer biology remains debatable. ANGPTL4 was identified as one of the most highly expressed genes in cancer patients (13) Despite the plethora of evidence implicating ANGPTL4 in cancer, the heterotypic role of ANGPTL4 in disease development remains unclear.

The present invention seeks to ameliorate at least one of the problems mentioned above.

### Summary

Accordingly a first aspect of the invention includes a method of modulating endothelial cell permeability by controlling the concentration or activity of Angiopoietin-like 4 (ANGPTL4) in the cell environment.

Another aspect of the invention includes a method for treating a patient to at least reduce endothelial cell permeability, which comprises the step of contacting the infection with an antagonist to cANGPTL4.

Another aspect of the invention includes a method comprising the steps of preparing an antibody using an adjuvant and a cANGPTL4; and administering the antibody to a patient suffering from vascular-related diseases.

Another aspect of the invention includes a composition comprising a therapeutically effective amount of a modulator of cANGPTL4 concentration.

Another aspect of the invention includes a use of the composition in the manufacture of a medicament for treating a patient suffering from vascular-related diseases.

Another aspect of the invention includes a method of determining the extent of endothelial cell permeability in a sample comprising the step of: measuring the amount of ANGPTL4 protein is in the sample.

Other aspects and advantages of the invention will become apparent to those skilled in the art from a review of the ensuing description, which proceeds with reference to the following illustrative drawings of preferred embodiments.

### Brief Description of the drawings

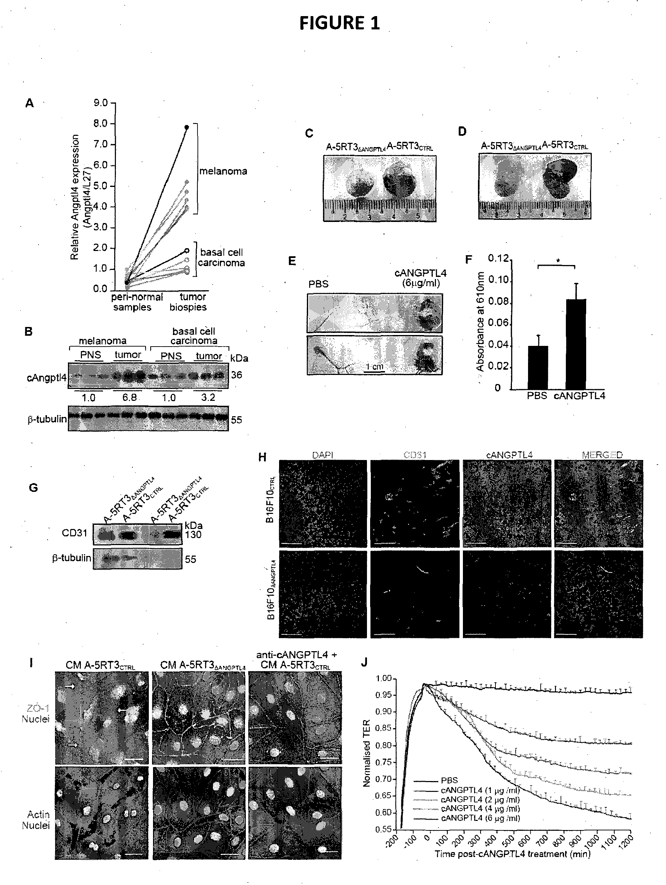

**Figure 1****: cANGPTL4 is elevated in metastatic tumors and disrupts endothelial junction integrity. (A, B)** Expression levels of cANGPTL4 mRNA (A) and protein (B) in basal cell carcinoma (BCCs) and metastatic melanoma paired with peri-tumor normal tissues as determined by qPCR and immunoblot, respectively (n=3). Data spots from same individual are linked by colored lines. Ribosomal protein L27 (L27) was used as a reference housekeeping gene. β-tubulin served as a loading and transfer control.**(C, D)** Evan's blue dye extravasation induced by either A-5RT3CTRL-, A-5RT3ΔANGPTL4- induced tumors of different (C) or similar (D) tumor volume. (A-5RT3CTRL 1098±422 mm3 v.s A-5RT3ΔANGPTL4 551±135mm3, *P* < 0.05, n=6) **(E, F)** Evan's blue dye extravasation induced by cANGPTL4 or PBS vehicle (G) and quantification (H) of extravasated Evan's blue dye by measurement of absorbance at 610nm. *, *P* < 0.05. **(G)** Immunodetection of CD31 in A-5RT3CTRL-, A-5RT3ΔANGPTL4-induced tumors. **(H)** Immunofluorescence staining for CD31 and cANGPTL4 in B16F10CTRL- and B16F10ΔANGPTL4-induced tumors. Scale bar: 100 µm. **(I)** Immunofluorescence staining for ZO-1 in a confluent HMVEC monolayer. Cells were treated with either CM from A-5RT3CTRL in the presence or absence of anti-cANGPTL4, from A-5RT3ΔANGPTL4 cells. HMVECs were counterstained with DAPI (blue) for nuclei and phalloidin (red) for actin stress fibers. Scale bar: 40 µm. White arrows indicate endothelial junction lesions. **(J)** Transendothelial electrical resistance (TER) analysis of confluent monolayer HMVECs treated with the indicated amounts of cANGPTL4. PBS served as a vehicle control. Data (means±S.D.) from 3 independent experiments.

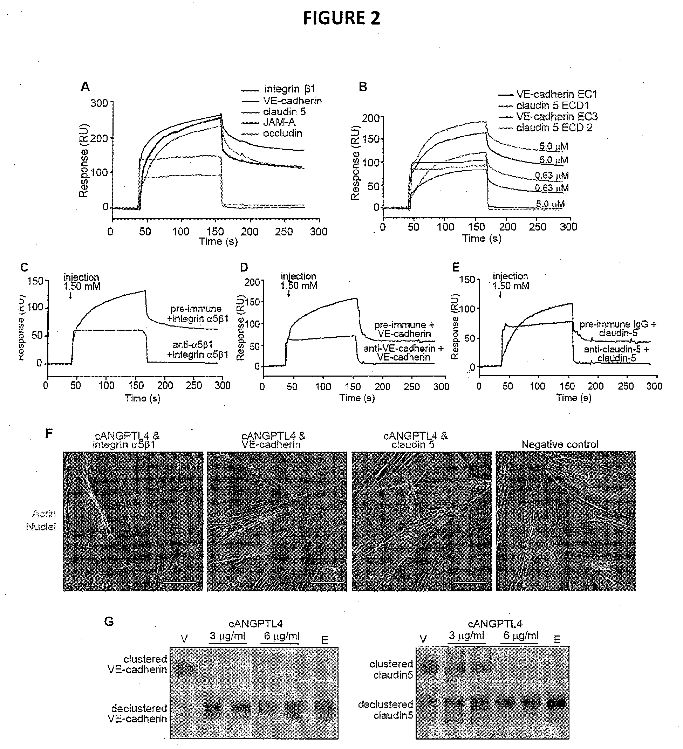

**Figure 2****: cANGPTL4 interacts with integrin α5β1, VE-cadherin and claudin-5. (A-E)** Representative sensorgrams showing binding profiles between immobilizedcANGPTL4 and integrin α5β1, VE-cadherin, claudin-5, occludin and junctional adhesion molecule A (JAM-A) (A); VE-cadherin extracellular repeat domain 1(EC1), claudin-5 first extracellular domain (ECD1), VE-cadherin EC3 and claudin-5 ECD2 (B); Integrin α5β1 (C),VE-cadherin (D) and claudin-5 (E) pre-blocked with either preimmune IgG or indicated cognate antibodies. **(F)** *In situ* proximity ligation assay (PLA) of cANGPTL4 and indicated binding partners. PLA signals are in red (Integrin 22.50±5.01 v.s VE-cadherin 16.43±2.46 v.s claudin-5 12.35±3.44 PLA spots/cell for 250 cells, n=3). Cells were counterstained with Alexa488- phallodin for actin stress fibers (green) and Hoechst dye for nuclei (blue). Negative control was performed in the absence of anti-ANGPTL4. Scale bar: 40 µm. **(G)** Immunodetection of VE-cadherin and claudin-5 from membrane extract of HMVECs treated with cANGPTL4 (3 or 6 µg/ml) for 3 h. Vehicle (v) is PBS, and E indicates treatment with 2.5 mM EDTA in PBS.

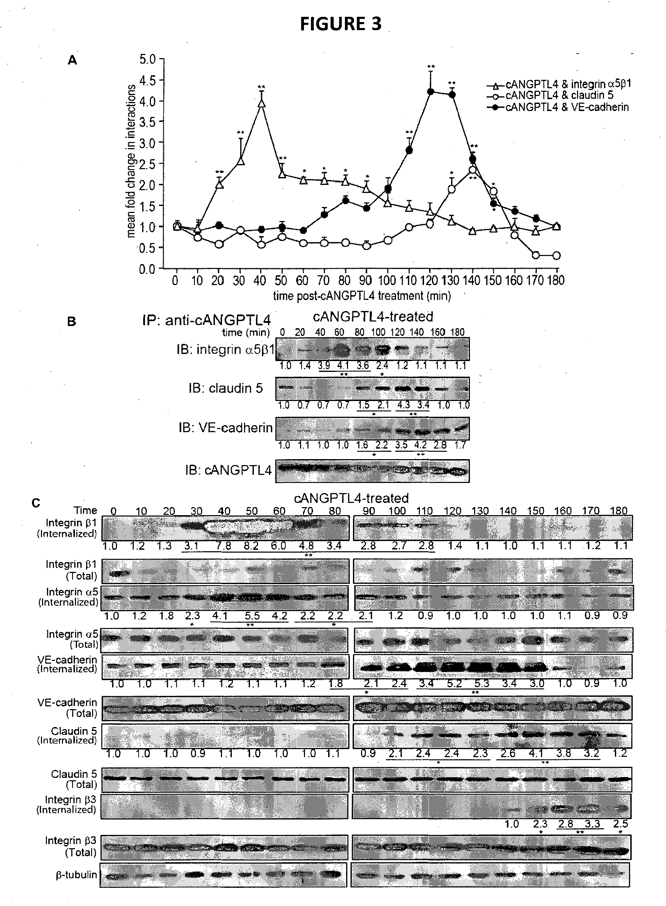

**Figure 3****: cANGPTL4 interacts with integrin α5β1, VE-cadherin and claudin-5 in a bimodal manner. (A)** *In situ* kinetic PLA detection of various complexes between cANGPTL4 and indicated binding partners in HMVECs treated with 6 µg/ml of cANGPTL4. Values (means±S.D.) represent mean fold change in the number of interactions compared to the zero time point, as determined from n=3 independent experiments (600 HMVECs) using BlobFinder. Experiments were terminated when microscopic lesions were observed at 180 min post-cANGPTL4 treatment. **(B)** Immunodetection of integrin α5β1, VE-cadherin and claudin-5 in anti-cANGPTL4 immunoprecipitates from total protein lysates of HMVECs treated with 6 µg/ml rhcANGPTL4. **(C)** Immunodetection of integrins β1, integrin α5, integrin β3, VE-cadherin and claudin-5 from total protein lysate versus internalized fraction of HMVECs treated with cANGPTL4. Protein lysates were collected every 10 minutes (0-180 minutes). Values below each band represent the mean fold change in protein expression level compared with the cognate zero time point (n=3). *, *P* < 0.05; **, *P* < 0.01.

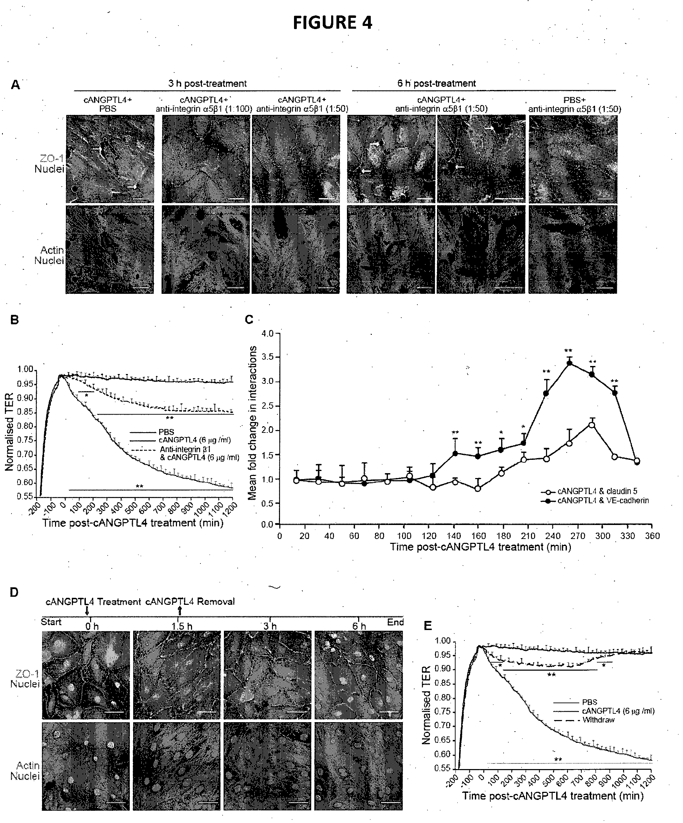

**Figure 4****: Inhibition of cANGPTL4:interacting partner complex formation delays**
   **endothelial disruption. (A, B and D, E)** Immunofluorescence staining for ZO-1 (A, D) and transendothelial electrical resistance (TER) measurement (B, E) in confluent HMVEC monolayer. Cells were treated with either 6 µg/ml of cANGPTL4 in the presence of increasing anti-integrin β1 concentrations (1:100, 1:50) (A, B) or 6 µg/ml of cANGPTL4 followed by removal of exogenous cANGPTL4 at 90 min (D, E). Treatments were for 3 or 6 h. HMVECs were counterstained with DAPI (blue) for nuclei and phalloidin (red) for actin stress fibers. Scale bar: 40 µm. White arrows indicate endothelial junction lesions.. Data (means±S.D.) from 3 independent experiments. *, *P* < 0.05; **, *P* < 0.01. **(C)** *In situ* kinetic PLA detection of cANGPTL4: indicated binding partner complexes in cANGPTL4-treated HMVECs in the presence of anti-integrin β1. Values (means±S.D.) represent mean fold change in the number of interactions compared to the zero time point, as determined from n=3 independent experiments (∼600 HMVECs) using BlobFinder. Experiments were terminated when microscopic lesions were observed (360 minutes post-cANGPTL4 treatment).

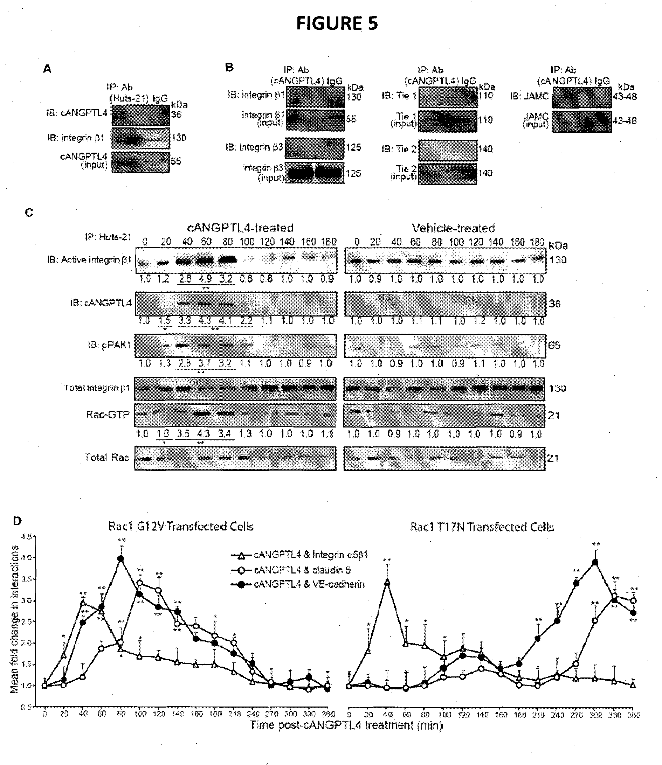

**Figure 5****: cANGPTL4-activated Integrin β1 mediates vascular permeability via activated Rac/PAK signaling axis. (A, B)** Immunodetection of indicated proteins in anti-Huts-21 (A) and anti-cANGPTL4 (B) immunoprecipitates from total protein lysates of rh-cANGPTL4-treated HMVECs (6 µg/ml). IgG immunoprecipitates serves as control. **(C)** Immunodetection of indicated proteins from anti-Huts-21 immunoprecitates of HMVECs treated with either vehicle (PBS) or rh-cANGPTL4. Total integrin □1, Rac1 and PAK served as controls. Protein lysates were collected every 20 minutes (0-180 minutes). Values below each band represent the mean fold change in protein expression level from three independent experiments compared with the cognate zero time point. *, *P* < 0.05; **, *P* < 0.01. **(D)** *In situ* kinetic PLA detection of cANGPTL4: indicated binding partner complexes in cANGPTL4-treated HMVECs transfected with either constitutive-active Rac1 G12V or dominant-negative Rac1 T17N. Values (means±S.D.) represent mean fold change in the number of interactions compared to the zero time point, as determined from n=3 independent experiments (∼600 HMVECs) using BlobFinder. *, *P* < 0.05; **, *P* < 0.01.

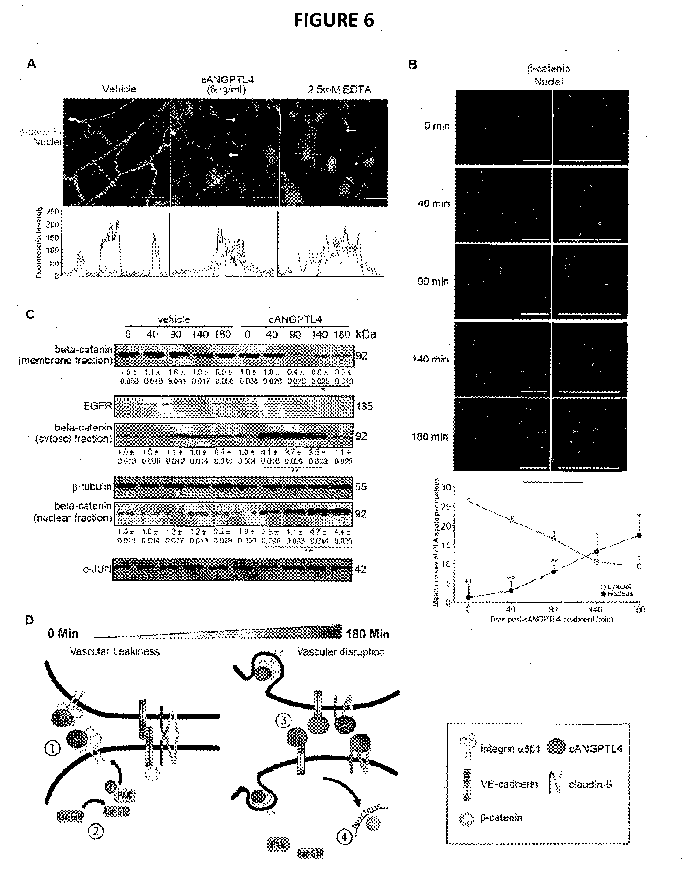

**Figure 6****: cANGPTL4:interacting partner complex formation triggers the nuclear translocation of β-catenin. (A)** Immunofluorescence staining for β-catenin (green) in a HMVEC monolayer treated with either 6 µg/ml of cANGPTL4 or 2.5mM EDTA (as positive control) for 3 h. HMVECs were counterstained with DAPI (blue) for nuclei. Scale bar: 40 µm. White arrows indicate endothelial junction lesions associated with reduced β-catenin staining. Representative fluorescence intensity plot of β-catenin and DAPI, indicated by the white dotted line across the nuclei, were quantified using Zen 2009 software (Carl Zeiss). **(B)** *In situ* PLA assay for β-catenin and quantification of the number of PLA spots per nuclei of cANGPTL4-treated HMVECs at the indicated time points. (0 min, 1.2±0.81; 40 min, 3±1.23; 90 min, 7.9±1.93; 140 min, 13.3±2.44; 180 min, 17.5±2.68) PLA signals (red) revealed translocation of β-catenin into nuclei at 140 min after treatment (∼250 HMVECs). Cells were counterstained with Hoechst dye for nuclei (blue). Scale bar: 40 µm. *, *P* < 0.05; **, *P* < 0.01. **(C)** Immunodetection of β-catenin in membrane, cytosol and nuclei fractions of HMVECs treated with either vehicle (PBS) or cANGPTL4. Protein lysates were collected at the indicated times. Values (means±S.D.) below each band represent the mean fold change in protein expression level compared with the cognate zero time point (n=3). *, *P* < 0.05; **, *P* < 0.01. **(D)** Schematic diagram of cANGPTL4-mediated disruption of endothelial junctions. (1) cANGPTL4:integrin α5β1 formation (30-50 min) coincides with (2) the activation of Rac-GTP and pPAK in ECs (40-60 min) and with vascular leakiness; (3) the interaction between cANGPTL4 with VE-cadherin and claudin-5 (120-140 min) disrupts intercellular contact formation, and stimulates (4) nuclear translocation of β-catenin (180 min).

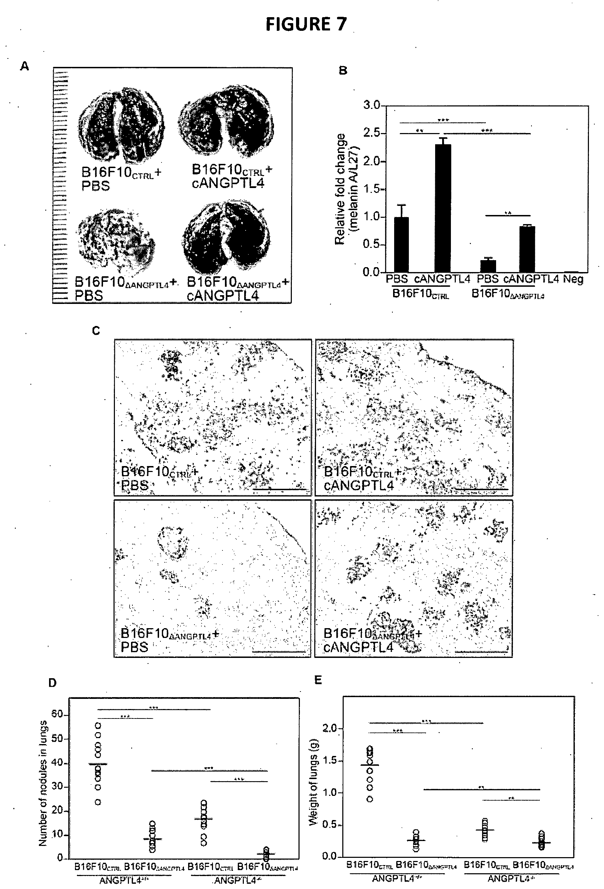

**Figure 7****: cANGPTL4 facilitates metastasis (A-C)** Representative macroscopic images of lungs (A); relative expression of melanin A (B); representative eosin stained images of lung sections(C), from (3mg/kg, thrice weekly). Black nodules in (C) indicate intravasated melanoma (n=5). Scale bar: 100 µm **(D, E)** Number of nodules (D) and weights of lungs (E) from wild type and ANGPTL4 knockout C57BL/6J mice intravenously injected with either 5 x 105 B16F10CTRL or B16F10ΔANGPTL4 cells (n=12). **, *P* < 0.01; ***, *P* < 0.001.

### Detailed Description

Here, we show that tumor-derived cANGPTL4 instigated the disruption of endothelial continuity by directly interacting with integrin α5β1, VE-cadherin and claudin-5 in a temporally sequential manner. Tumor-derived cANGPLT4 and recombinant human cANGPTL4 (rh-cANGPTL4) increased vascular permeability *in vitro* and *in vivo.* Using *ANGPTL4*-knockout and wild-type mice injected with either control or ANGPTL4-knockdown tumors confirmed that cANGPTL4 induced vascular leakiness and facilitated lung metastasis in mice. cANGPTL4 binds to and activates integrin α5β1-mediated PAK/Rac signaling which weakened EC-EC contacts and increased vascular leakiness. cANGPTL4 associated with the adhesive domains of VEcadherin and claudin-5, resulting in their declustering and internalization, translocation of β-catenin to the nucleus and a leaky endothelium. These results identify cANGPTL4 as a novel upstream mediator of endothelium permeability and a potential target in the treatment of cancer and other vascular-related pathologies.

As such a method of modulating endothelial cell permeability by controlling the concentration or activity of Angiopoietin-like 4 (ANGPTL4) in the cell environment is contemplated.
Cell permeability refers to "Paracellular permeability" between two cells, as well as "transcellular" through the cell.

In one embodiment the cell environment is *in vivo.* In another embodiment the cell environment is *in vitro.*

Where modulation of ANGPTL4 includes increasing the concentration or activity of Angiopoietin-like 4 (ANGPTL4) and/or cANGPTL4 then endothelial cell permeability is increased.

Alternatively, where modulation of ANGPTL4 includes removing, degrading or neutralising the concentration or activity of Angiopoietin-like 4 (ANGPTL4) and/or cANGPTL4 then endothelial cell permeability is minimized reduced or inhibited. In one embodiment the Angiopoietin-like 4 (ANGPTL4) and/or cANGPTL4 is removed, degraded or neutralised by a cANGPT4L specific antibody as described herein. In one embodiment the antibody is catalytic. In another embodiment the Angiopoietin-like 4 (ANGPTL4) and/or cANGPTL4 is removed, degraded or neutralised by an siRNA or other antisense technology specific to ANGPTL4 polypeptide expression

Another aspect includes a method for treating a patient to at least reduce endothelial cell permeability, which comprises the step of contacting the cell with an antagonist to cANGPTL4. Preferably, the antagonist is an antibody to cANGPTL4. In one embodiment the antibody is a neutralizing antibody to cANGPTL4. Preferably, the antagonist interferes with increased endothelial cell permeability. Preferably, the antagonist comprises a compound that engages an cANGPTL4 domain of ANGPTL4 preventing increased endothelial cell permeability.

Another aspect includes a method comprising the steps of preparing an antibody using an adjuvant and a cANGPTL4 polypeptide; and administering the antibody to a patient suffering from vascular-related disease.

In one embodiment the antibody is formed in the patient suffering from vascular-related disease by injecting the adjuvant and a cANGPTL4 polypeptide as the antigen directly into the patient in the form of a vaccine.

Preferably the vascular-related diseases are selected from the group consisting of inflammatory edema, diabetes, atherosclerosis.

In one embodiment the vascular-related diseases is cancer metastasis.In this embodiment the method may further comprise administering an anticancer agent to the patient.

Another aspect includes a composition comprising a therapeutically effective amount of a modulator of cANGPTL4 concentration.

In one embodiment the modulator is an antagonist to cANGPTL4. Preferably, the antagonist is an antibody to cANGPTL4 such as a catalytic antibody to cANGPTL4. Preferably the composition comprises a compound that engages a cANGPTL4 domain of ANGPTL4 preventing increased endothelial cell permeability.
In one embodiment the composition is for use as a medicament for treating a patient suffering from vascular-related diseases. Vascular-related diseases may include inflammatory edema, diabetes, atherosclerosis, or cancer metastasis

Another aspect of the invention includes a composition comprising a therapeutically effective amount of a modulator of cANGPTL4 concentration.

Another aspect includes a use of the composition in the manufacture of a medicament for treating a patient suffering from vascular-related diseases.The vascular-related diseases may include or be selected from the group consisting of inflammatory edema, diabetes, atherosclerosis, and cancer metastasis.

In the embodiment where the vascular-related disease is cancer metastasis the composition may include an anticancer agent to the patient.

Another aspect includes a method of determining the extent of endothelial cell permeability in a sample comprising the step of: measuring the amount of ANGPTL4 protein is in the sample. Preferably, the method may further comprise the step of determining the patient's treatment according to whether the amount of ANGPTL4 protein is indicative of increased endothelial cell permeability.

### Antibodies

An antibody may include an immunoglobulin that specifically binds to the C terminal of the ANGPTL4 protein.The immunoglobulin may comprises an immunoglobulin heavy chain and/or an immunoglobulin light chain.

Preferably, the immunoglobulin is an IgG1 kappa immunoglobulin.most preferably; the immunoglobulin comprises a human IgG1 constant region within a heavy chain of said immunoglobulin and a human constant region within a light chain of said immunoglobulin. The immunoglobulin may comprises fully or partially human framework regions within the variable domain of said heavy chain and within the variable domain of said light chain. The immunoglobulin may comprises murine framework regions within the variable domain of said heavy chain and within said light chain.
The immunoglobulin wherein said immunoglobulin is conjugated to an agent selected from the group consisting of a therapeutic agent, a prodrug, a peptide, a protein, an enzyme, a virus, a lipid, a biological response modifier, a pharmaceutical agent, and PEG.

### Polyclonal Antibodies

The antibodies may comprise polyclonal antibodies. Methods of preparing polyclonal antibodies are known to the skilled artisan. Polyclonal antibodies can be raised in a mammal, for example, by one or more injections of an immunizing agent and, if desired, an adjuvant.
Typically, the immunizing agent and/or adjuvant will be injected in the mammal by multiple subcutaneous or intraperitoneal injections. The intensity of the response is determined by several factors including the size of the immunogen molecule, its chemical characteristics, and how different it is from the animal's own proteins. Most natural immunogens are proteins with a molecular weight above 5 kDa that come from sources phylogenically far removed from the host animal (i.e., human proteins injected into rabbits or goats). It is desirable to use highly purified proteins as immunogens, since the animal will produce antibodies to even small amounts of impurities present as well as to the major component. The antibody response increases with repeated exposure to the immunogen, so a series of injections at regular intervals is needed to achieve both high levels of antibody production and antibodies of high affinity.
To the extent that the antagonist is an antibody that engage the c terminal of the fibrinogen-like fragment of ANGPTL4 preventing cell proliferation, the immunogen will be an selected from amino acids comprising the c terminal of the fibrinogen-like fragment of ANGPTL4. Preferably, the amino acid sequence will be selected from the region of about 186 - 406 in the human ANGPTL4 protein or about 190 - 410 in the mouse ANGPTL4 protein. Sequences of at least 5, 6, 7, 8, 9, 10, 15, 20, 25, 30 amino acids from this region will generally be used to generate those antibodies. Desirably, the sequence selected will generate an antibody that specifically interferes with binding of ANGPTL4 to components of intracellular signaling pathways.
Not all immunogenic molecules will however generate the level of antibody desired. To increase the intensity of the immune response immunogens are combined with complex mixtures called adjuvants. Adjuvants are a mixture of natural or synthetic compounds that, when administered with antigens, enhance the immune response. Adjuvants are used to (1) stimulate an immune response to an antigen that is not inherently immunogenic, (2) increase the intensity of the immune response, (3) preferentially stimulate either a cellular or a humoral response (i.e., protection from disease versus antibody production). Examples of adjuvants which may be employed include Freund's complete adjuvant and MPL-TDM adjuvant (monophosphoryl Lipid A, synthetic trehalose dicorynomycolate). A more extensive discussion of adjuvants and their use in immunization protocols is given in Immunology Methods Manual, vol. 2, I. Lefkovits, ed., Academic Press, San Diego, CA, 1997, ch. 13. Immunology Methods Manual is available as a four volume set, (Product Code Z37,435-0); on CD-ROM, (Product Code Z37,436-9); or both, (Product Code Z37,437-7)
If the immunogen is still unable to generate an acceptable response, it may be conjugated to a carrier protein that is more immunogenic. Small molecules such as drugs, organic compounds, and peptides and oligosaccharides with a molecular weight of less than 2-5 kDa like, for example, small segments if ANGPTL4 such as those with a fibrinogen-like fragment in their structure, may not be immunogenic, even when administered in the presence of adjuvant. In order to generate an immune response to these compounds, it is necessary to attach them to a protein or other compound, termed a carrier that is immunogenic. When attached to a carrier protein the small molecule immunogen is called a hapten. Haptens are also conjugated to carrier proteins for use in immunoassays. The carrier protein provides a means of attaching the hapten to a solid support such as a microtiter plate or nitrocellulose membrane. When attached to agarose they may be used for purification of the anti-hapten antibodies. They may also be used to create a multivalent antigen that will be able to form large antigen-antibody complexes. When choosing carrier proteins, remember that the animal will form antibodies to the carrier protein as well as to the attached hapten. It is therefore relevant to select a carrier protein for immunization that is unrelated to proteins that may be found in the assay sample. If haptens are being conjugated for both immunization and assay, the two carrier proteins should be as different as possible. This allows the antiserum to be used without having to isolate the anti-hapten antibodies from the anti-carrier antibodies.
Where the immunizing agent is a fibrinogen-like fragment segment such as from the c terminal preferably the fibrinogen-like fragment segment is conjugated to a protein known to be immunogenic in the mammal being immunized.
Examples of such immunogenic proteins include but are not limited to keyhole limpet hemocyanin (KLH), serum albumin, bovine thyroglobulin, soybean trypsin inhibitor, and a toxoid, for example tetanus toxoid.
KLH is a respiratory protein found in molluscs. Its large size makes it very immunogenic, and the large number of lysine residues available for conjugation make it very useful as a carrier for haptens. The phylogenic separation between mammals and molluscs increases the immunogenicity and reduces the risk of cross-reactivity between antibodies against the KLH carrier and naturally occurring proteins in mammalian samples.
KLH is offered both in its native form, for conjugation via amines, and succinylated, for conjugation via carboxyl groups. Succinylated KLH may be conjugated to a hapten containing amine groups (such as a peptide) via cross-linking with carbodiimide between the newly introduced carboxyl groups of KLH and the amine groups of the hapten.
Protocols for conjugation of haptens to carrier proteins are known.
The immunization protocol may be selected by one skilled in the art without undue experimentation. Protocols for preparing immunogens, immunization of animals, and collection of antiserum are also known.

### Monoclonal Antibodies

The antibodies may, alternatively, be monoclonal antibodies. Monoclonal antibodies may be prepared using hybridoma methods, such as those known. In a hybridoma method, a mouse, hamster, or other appropriate host animal, is typically immunized with an immunizing agent as described above to elicit lymphocytes that produce or are capable of producing antibodies that will specifically bind to the immunizing agent. Alternatively, the lymphocytes may be immunized *in vitro.* Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell. Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.
Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, Calif. and the American Type Culture Collection, Manassas, Va. Human myeloma and mouse-human heteromyeloma cell lines also have been described for the production of human monoclonal antibodies.
The culture medium in which the hybridoma cells are cultured can then be assayed for the presence of monoclonal antibodies directed against ANGPTL4 and/or the the c terminal of ANGPTL4 or any of the sequences SEQ ID No. 1 to 3.
After the desired hybridoma cells are identified, the clones may be subcloned by limiting dilution procedures and grown by standard methods. Suitable culture media for this purpose include, for example, Dulbecco's Modified Eagle's Medium and RPMI-1640 medium. Alternatively, the hybridoma cells may be grown *in vivo* as ascites in a mammal.
The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.
The monoclonal antibodies may also be made by recombinant DNA methods, such as those known.
The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain cross-linking. Alternatively, the relevant cysteine residues are substituted with another amino acid residue or are deleted so as to prevent cross-linking.
*In vitro* methods are also suitable for preparing monovalent antibodies. Digestion of antibodies to produce fragments thereof, particularly, Fab fragments, can be accomplished using routine techniques known in the art.

### Human and Humanized Antibodies

The antibodies of the invention may further comprise humanized antibodies or human antibodies. Humanized forms of non-human (e.g., murine) antibodies are chimeric immunoglobulins, immunoglobulin chains or fragments thereof (such as Fv, Fab, Fab', F(ab')₂ or other antigen-binding sub-sequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin.
Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain. Humanization can be essentially performed following the methods known by substituting rodent CDRs or CDR sequences for the corresponding sequences of a human antibody. Accordingly, such "humanized" antibodies are chimeric antibodies, wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.
Human antibodies can also be produced using various techniques known in the art, including phage display libraries. Similarly, human antibodies can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire.

### Bispecific Antibodies

Bispecific antibodies are monoclonal, preferably human or humanized, antibodies that have binding specificities for at least two different antigens. In the present case, one of the binding specificities is for ANGPTL4 and/or a segment of ANGPTL4 comprising portions of the c terminal of the fibrinogen-like fragment of ANGPTL4, the other one is for another compound interacting with ANGPTL4. Methods for making bispecific antibodies are known in the art. Traditionally, the recombinant production of bispecific antibodies is based on the co-expression of two immunoglobulin heavy-chain/light-chain pairs, where the two heavy chains have different specificities.

### Heteroconjugate Antibodies

Heteroconjugate antibodies are also within the scope of the present invention. Heteroconjugate antibodies are composed of two covalently joined antibodies. Such antibodies have, for example, been proposed to target immune system cells to unwanted cells. It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and others known to a person skilled in the art.

### Immunoconjugates

The invention also pertains to immunoconjugates comprising an antibody conjugated to a cytotoxic agent such as a chemotherapeutic agent, toxin (e.g., an enzymatically active toxin against hemagglutinin), or a radioactive isotope (i.e., a radioconjugate).
Conjugates of the antibody and cytotoxic agent are made using a variety of bifunctional protein-coupling agents such as N-succinnimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis(p-azidobenzoyl)hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene).

### Method of treatment and or use of the antibodies of the invention

The present invention also provides a method of treating a patient to at least affect a proliferative disorder, which comprises the step of: contacting a cell with an antagonist such as (a) an antibody specific to angiopoietin like 4 protein (ANGPTL4) or (b) an antibody specific to the C terminal region of angiopoietin like 4 protein (ANGPTL4). Preferably, the antagonist interferes with cell proliferation by means that neutralize angiopoietin like 4 protein (ANGPTL4) expression.
An alternative form of the present invention resides in the use of an antibody specific angiopoietin like 4 protein (ANGPTL4) or an antibody specific to the C terminal region of angiopoietin like 4 protein (ANGPTL4) for the treatment of cancer, preferably the use at least affects cell proliferation.
Cancer may include, all types of known tumors that exhibit over expression of angiopoietin like 4 protein (ANGPTL4). Cancer metastasis includes cells exhibiting vascular leakiness from increase cell permeability. Cell proliferating or tumor refers to cells that are growing uncontrollably.
"Treatment" and "treat" and synonyms thereof refer to therapeutic treatment wherein the object is to prevent or slow down (lessen) a tumor or reduce vascular leakiness from increase cell permeability. Treatment may include prophylactic passive immunization or immunotherapy treatment of a patent. Those in need of such treatment include those with a proliferative disorder.
As used herein a "therapeutically effective amount" of a compound will be an amount of active agent that is capable of preventing or at least slowing down (lessening) cell vascular leakiness from increase cell permeability. Dosages and administration of an antagonist of the invention in a pharmaceutical composition may be determined by one of ordinary skill in the art of clinical pharmacology or pharmacokinetics. An effective amount of the antagonist to be employed therapeutically, for example an antibody, will depend, for example, upon the therapeutic objectives, the route of administration, and the condition of the mammal. Accordingly, it will be necessary for the therapist to titer the dosage and modify the route of administration as required to obtain the optimal therapeutic effect. A typical daily dosage might range from about 10 ng/kg to up to 100 mg/kg of the mammal's body weight or more per day, preferably about 1 µg/kg/day to 10 mg/kg/day. Doses may include an antibody amount anywhere in the range of 0.1 to 20 mg/kg of bodyweight or more preferably 1, 5, 10 mg/kg of bodyweight.

### Compositions of the Invention

Thus, the present invention also relates to compositions including pharmaceutical compositions comprising a therapeutically effective amount of (a) an antibody specific to angiopoietin like 4 protein (ANGPTL4) and, or (b) an antibody specific to the C terminal region of angiopoietin like 4 protein (ANGPTL4). As used herein a compound will be therapeutically effective if it is able to affect cell permeability. Pharmaceutical forms of the invention suitable for injectable use, include sterile aqueous solutions such as sterile phosphate-buffered saline (where water soluble) or dispersions and sterile powders for the extemporaneous preparation of sterile injectable solutions and or one or more carrier. Alternatively, injectable solutions may be delivered encapsulated in liposomes to assist their transport across cell membrane. Alternatively or in addition such preparations may contain constituents of self-assembling pore structures to facilitate transport across the cellular membrane. It must be stable under the conditions of manufacture and storage and must be preserved against the contaminating/destructive action of microorganisms such as, for example, bacteria and fungi.
The carrier can be a solvent or dispersion medium containing, for example, water, ethanol, polyol (for example, glycerol, propylene glycol and liquid polyethylene glycol, and the like), suitable mixtures thereof, and vegetable oils. The proper fluidity can be maintained, for example, by the use of a coating such as, for example, lecithin, by the maintenance of the required particle size in the case of dispersion and by the use of surfactants. Preventing the action of microorganisms in the compositions of the invention is achieved by adding antibacterial and/or antifungal agents, for example, parabens, chlorobutanol, phenol, sorbic acid, thimerosal and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin. Sterile injectable solutions are prepared by incorporating the active compounds in the required amount in the appropriate solvent with several of the other ingredients enumerated above, as required, followed by filtered sterilization. Generally, dispersions are prepared by incorporating the various sterilized active ingredient into a sterile vehicle which contains the basic dispersion medium and the required other ingredients from those enumerated above. In the case of sterile powders for the preparation of sterile injectable solutions, the preferred methods of preparation are vacuum drying and freeze-drying, to yield a powder of the active ingredient plus any additional desired ingredient from previously sterile-filtered solution thereof. The active ingredient may be held within a matrix which controls the release of the active agent. Preferably, the matrix comprises a substance selected from the group consisting of lipid, polyvinyl alcohol, polyvinyl acetate, polycaprolactone, poly(glycolic)acid, poly(lactic)acid, polycaprolactone, polylactic acid, polyanhydrides, polylactide-co-glycolides, polyamino acids, polyethylene oxide, acrylic terminated polyethylene oxide, polyamides, polyethylenes, polyacrylonitriles, polyphosphazenes, poly(ortho esters), sucrose acetate isobutyrate (SAIB), and combinations thereof and other polymers Preferably, the matrix sustainedly releases the antibody.
Pharmaceutically acceptable carriers and/or diluents may also include any and all solvents, dispersion media, coatings, antibacterials and/or antifungals, isotonic and absorption delaying agents and the like. The use of such media and agents for pharmaceutical active substances is well known in the art. Except insofar as any conventional media or agent is incompatible with the active ingredient, use thereof in the therapeutic compositions is contemplated.

### Polypeptides

The term "polypeptide" refers to a polymer of amino acids and its equivalent and does not refer to a specific length of the product; thus, peptides, oligopeptides and proteins are included within the definition of a polypeptide. This term also does not refer to, or exclude modifications of the polypeptide, for example, glycosylations, acetylations, phosphorylations, and the like. Included within the definition are, for example, polypeptides containing one or more analogs of an amino acid (including, for example, natural amino acids, etc.), polypeptides with substituted linkages as well as other modifications known in the art.

Our tissue microarray analysis revealed an elevated ANGPTL4 expression in up to 40 known human epithelial tumor types, and its expression increased as tumors progressed from a benign state to a metastatic state. We have identified tumor-derived ANGPTL4 as a novel player in redox cancer biology and confers anoikis resistance to tumors via autocrine adhesion mimicry. Tumor-derived ANGPTL4 interacts with integrins, hijacks integrin-mediated signaling to maintain an elevated, oncogenic O₂⁻:H₂O₂ ratio and, therefore, promotes cell survival and tumor growth. Importantly, the suppression of ANGPTL4, either by RNAi or immunosuppression with a monoclonal antibody, modulated intracellular ROS generation to attenuate tumor growth associated with enhanced apoptosis *in vitro* and *in vivo.* The elevated expression of ANGPTL4 in many metastatic cancers also implicated a role of ANGPTL4 in metastasis through lymphovascular invasion. Importantly, ANGPTL4 is distinct from VEGF, as it does not bind to VEGR receptor. Furthermore, VEGF knockout mice are embryonic lethal compare to ANGPTL4 knock-out mice, indicating a distinct role of ANGPTL4. We showed that tumor-derived Angptl4 can induce vascular leakiness and facilitates tumor cells dissemination. In this instance, antagonists against ANGPTL4, such as an antibody or siRNA, will inhibits metastasis and identify ANGPTL4 as novel target that is distinct from VEGF.

Recombinant ANGPTL4 to induce vascular leakiness will aid diabetic wound repair. ANGPTL4 functions both as a matricellular protein to accelerate cell migration, the increased vascular leakiness allows efflux of inflammatory cells and bone-marrow derived precursors into the site of injury accelerate wound repair. We have demonstrated that through interaction with matrix proteins, integrin β1 and integrin β5, cANGPTL4 facilitates cell migration and wound healing, both of which are processes highly reminiscent of metastasis.

### Examples

### Materials and methods

### Antibodies.

Antibodies for PAK1 and pPAK1 (Ser199/Ser204) (Cell Signaling, USA), integrin β1 [JB1A], α5β1[JBS5], β3[2008] and c-Jun (Millipore, USA); CD29/activated integrin β1 [HUTS-21] (BD, USA); β-tubulin, β-catenin [12F7], EGFR[1005] (Santa Cruz Biotechnologies, USA); VE-Cadherin [BV9], Claudin-5 (Abcam, USA); CD31 (DAKO, Denmark); Occludin (Invitrogen, USA); ZO-1(Zymed Laboratories, USA); Tie 1, Tie 2, JAM-C, β-tubulin, horseradish peroxidase (HRP)-conjugated goat anti-mouse, HRP-conjugated goat anti-rabbit, HRP-conjugated donkey anti-goat (Santa Cruz Biotechnologies, USA); mouse monoclonal anti-human cANGPTL4 mAb4A11 H5 and rabbit polyclonal anti-human cANGPTL4 were produced in-house16; Secondary antibodies Alexa Fluor 488-conjugated goat anti-rabbit IgG and Alexa Fluor 594- conjugated goat anti-mouse IgG were also used (Molecular Probes, USA).

### Cell Cultures.

Primary human microvascular endothelial cells (HMVECs; Lonza, Switzerland) were cultured in EndoGRO-MV-VEGF medium (Millipore, USA) in a humidified atmosphere of 5% CO2 at 37 □C. The culture surface was precoated with 0.1% gelatin in PBS. All other cell lines were cultured in DMEM supplemented with 10% FBS. Conditioned medium (CM) was obtained from 1 x 105 tumor cells grown in 1 ml of serum-free medium for three days.

### Transient transfections assay.

Transfections of HMVECs were carried out as per manufacturer's protocol (Promega, USA) with either constitutive-active Rac1 G12V or dominant-negative Rac1 T17N.

### Expression and purification of recombinant cANGPTL4 proteins.

The expression and purification of full-length ANGPTL4 and recombinant cANGPTL4 were performed as previously described17,18.

### In Vivo Tumorigenicity and Miles Vascular Permeability Assay.

Six-week-old BALB/c athymic female nude mice (20-22 g) were injected either cANGPTL4 (6 µg/ml) or 0.9% saline buffer was injected intradermally at adjacent locations on the back. To determine vascular permeability, tumor-bearing mice received an intravenous injection of Evans blue dye. Twenty min later, the mice were sacrificed and Evans blue extracted from the tumor and normal muscle as described in Miles vascular permeability assay. 19 The extravasated dye was extracted using formamide and the amount was quantified by measuring absorbance at 610nm. To determine the tumor vascular volume, 2MDa FITC conjugated dextran (10 mg/kg) was also injected intravenously for 20 min. Fluorescence readings was obtained using Nanodrop 3300 Fluorospectrometer. For another set of experiments, mice were injected subcutaneously at the interscapular region with either 2 × 106 or 8 × 106 cells (A-5RT3CTRL or A-5RT3ΔANGPTL4). Same procedures were carried out in C57B/L6J wild-type and ANGPTL4-knockout mice using B16F10CTRL or B16F10 ΔANGPTL4 cells. Injection sites were rotated to avoid site bias. The injected tumor cells were allowed to grow for eight weeks.

### In Vivo Metastasis Assay.

Wild type and ANGPTL4-knockout C57BL/6J mice were injected intravenously with either 5 x 105 B16F10CTRL or B16F10ΔANGPTL4. For cANGPTL4 treatment, wild type C57BL/6J mice i.v injected with either 2 x 106 B16F10CTRL or B16F10ΔANGPTL4 cells were treated with PBS or cANGPTL4 (3 mg/kg) thrice weekly. After 3 weeks, the mice were sacrificed and the lungs were harvested for further analyses. Total metastatic burden were quantified by RT-PCR of melanin A. Total mRNA were extracted from lungs and reverse-transcribed as described previously. 17,18

### Proximity Ligation Assay (PLA).

PLA was carried out according to the manufacturer's protocol (Olink Bioscience, Sweden) using indicated pairs of antibodies. Images were taken using a Zeiss LSM 710 confocal microscope with a 63x objective and ZEN 2009 software. PLA signals were quantified using BlobFinder software.20

### Transendothelial Electrical Resistance (TER) measurement.

TER was measured using an electrical cell-substrate impedance sensing system (ECIS, Applied BioPhysics). HMVECs were seeded on sterile 8-well gold-plated electrode arrays precoated with fibronectin at 2 x 105 cells/well and allowed to adhere and spread for 4 h at 37 °C. Data from the electrical resistance experiments were obtained over the experimental time course at 5 min intervals. Confluent HMVEC monolayers that had stable TERs for 1 h preceding administration of indicated treatments were used. As cells adhered and spread over the microelectrode, TER increased, whereas cell retraction, rounding or loss of adhesion was reflected by a decrease in TER. Resistance values for each microelectrode were normalized as the ratio of measured resistance to baseline resistance and plotted as a function of time.

### Internalization assay.

Confluent HMVECs monolayers were treated with either PBS or cANGPTL4 (6 µg/ml) for 3 h. At indicated time point, internalization assay was carried out as previously described. 17

### Immunofluorescence staining.

Disrupted tight junctions were visualized by immunofluorescence staining for zona occludens-1 (ZO-1). HMVECs were fixed with 4% paraformaldehyde for 15 min, permeablized with 0.2% Triton-X 100 for 15 min and blocked with 2% bovine serum albumin (BSA) containing 0.1% Triton-X 100 in a humid chamber for 1 h at room temperature. Cells were incubated overnight at 4 °C with antihuman- ZO-1 antibodies (1:100) in 0.2% BSA. Following two washes in PBS, cells were incubated for 1 h at room temperature with Alexa488-secondary antibodies (1:250). Cells were counterstained with Alexa594-phalloidin for F-actin and DAPI for nuclei. Immunostainings performed without primary antibodies served as negative controls. For β-catenin staining, cells were fixed with 4% paraformaldehyde containing 5% sucrose for 15 min, permeablized with 0.5% Triton-X 100 in PBS for 4 min and blocked in 5% normal goat serum (NGS) with 0.1% Triton-X 100. Cells were incubated overnight at 4 oC with anti-human β-catenin antibodies (1: 200) in 3% NGS, followed by Alexa594- secondary antibodies. Images were acquired using Zeiss LSM 710 confocal microscope with a 63x objective and ZEN 2009 software.

### Surface Plasmon Resonance (SPR).

SPR was carried out as previously described17,18 using a BIAcore 2000 system (BIAcore, Uppsala, Sweden). Six concentrations (0.16, 0.32, 0.63, 1.25, 2.50 and 5.0 µM) of recombinant integrin β1, first extracellular domain of VE-cadherin (ECD1), extracellular repeat 1 domain of VE-cadherin (EC1), occludin or JAM-A were used. Integrin was expressed in Drosophila S2 cells17 and various extracellular domains of VE-cadherin and claudin-5 purchased from Abnova. Global fitting of the SPR data was performed as previously described to determine the KD. Preinjection or preincubation with the indicated antibodies or preimmune IgG was performed to determine specific interactions. Each sensorgram was corrected by subtracting a sensorgram obtained from a reference flow cell with no immobilized protein. The Rmax value was determined to be 283.1 resonance units using anticANGTL4 antibodies against the immobilized cANGPTL4. Values are mean±S.D. (n=3)

### Statistical Analysis.

Statistical analyses were performed using two-tailed Mann-Whitney tests with SPSS software. All statistical tests were two-sided. p value of ≤ 0.05 is considered significant.

### Elevated cANGPTL4 expression in human tumors.

To examine the expression profile of cANGPTL4 in tumors, we performed immunofluorescence using anti-cANGPTL4 antibody on two human tumor tissue arrays that covered benign, malignant and metastatic tumors originating from various anatomic sites. cANGPTL4 was elevated in all epithelial tumor samples compared to the corresponding normal tissue samples (Figures. S1A-C). Although fluorescence signals varied among different tumor types, they clearly showed that elevated cANGPTL4 was a common feature of tumors. Consistent with the abovementioned observations, cANGPTL4 protein level was elevated in all cancer cell lines when compared to non-tumorigenic lines (HaCaT and BJ) (Figure S1D). Interestingly,we observed relatively higher cANGPTL4 levels in metastatic than in non-metastatic cancer cell lines, suggesting a role of cANGPTI4 in tumor metastasis.

To further corroborate the role of cANGPTL4 in metastasis clinically, we determined cANGPTL4 mRNA and protein levels in six human basal cell carcinoma (benign) and melanoma (metastatic) biopsies by quantitative real-time PCR (qPCR) and immunoblot analyses. We observed significant upregulation of cANGPTL4 mRNA and protein levels in these epithelial cancers when compared with their cognate peri-tumor normal samples (PNSs). Notably, the melanomas expressed higher levels of cANGPTL4 mRNA and protein when compared to benign basal cell carcinoma (Figure 1A, B). Similar trend was observed when comparing cANGPTL4 protein levels of fine-needle aspirates (FNA) from three breast cancer patients to four human metastatic tumor lines (A-5RT3, MDA-MB231, MCF-7 and HT29) and two non-tumorigenic lines (HaCaT and BJ). The mean cANGPTL4 protein concentrations were 12.4 µg/ml (FNA), 5.9 µg/ml (metastatic lines) and 0.8 µg/ml (non-tumorigenic lines) (Figure S1E). These data suggest that epithelial tumors secrete elevated levels of cANGPTL4, which is consistent with our recent findings showing that tumor tissue expressed high levels of cANGPTL4 compared to basal level expression in the surrounding stroma. Altogether, our findings implicate a role for cANGPTL4 in cancer metastasis.

### cANGPTL4 increased vascular leakiness in in vivo tumors.

We next explored the role of cANGPTL4 in tumorigenesis. Metastatic human squamous cell carcinoma cell line A- 5RT3 and murine melanoma B16F10 were transduced with either scrambled (control) siRNA (A-5RT3CTRL or B16F10CTRL) or siRNA against ANGPTL4 (A-5RT3ΔANGPTL4 or B16F10ΔANGPTL4) (Figure S2A) to suppress endogenous ANGPTL4, as previously described.16,18 The injection of A-5RT3CTRL cells into immunodeficient mice induced large primary tumors at week 8, but A-5RT3ΔANGPTL4-induced tumors displayed a 30% lower tumor volume (A-5RT3CTRL 1098±422 mm3 v.s A-5RT3ΔANGPTL4 551±135mm3, P<0.05, n=6) (Figure 1C). Miles assays revealed decreased vascular leakiness in A- 5RT3ΔANGPTL4-induced tumors (∼5 fold lower), as evidenced by decreased extravasation of Evans blue dye (Figure 1D, Table 1). Differences in vascular volume among the tumor types were accounted for when determining tumor vascular permeability (Table 1). This differential vascular permeability was independent of the tumor volume, as observed in A-5RT3CTRL-and A-5RT3ΔANGPTL4-induced tumors (Figure 1D). Furthermore, none of the mice injected with A-5RT3ΔANGPTL4 developed distant metastases to the lymph nodes, whereas 83% of mice injected with A-5RT3CTRL showed metastases (Figure S2B).

Notably, rh-cANGPLT4 was sufficient to induce vascular leakiness when compared to the PBS control (rh-cANGPTL4 0.084±0.014 v.s PBS 0.04 ± 0.01, *P*<0.05, n=3) (Figures 1E, F). Consistent to the increased vascular volume in A-5RT3CTRL- and B16F10CTRLinduced tumors, the expression of EC marker CD31 was elevated when compared with their cognate ANGPTL4-deficient tumors (Figure 1G, H). Dual immunostaining for CD31 and cANGPTL4 also confirmed that epithelial tumors were the major producer of cANGPTL4, consistent with earlier findings using laser-captured microdissected samples (Figures 1H). Hence, these observations *in vivo* prompted us to investigate the molecular mechanism of cANGPTL4-induced endothelial permeability.

**Table 1. ANGPTL4 increases vascular permeability and vascular volume**

| **Tumor (n=15) OD610 Evans blue/g Fluorescent unit (FITC)/g Ratio Vascular** | | | | |
|---|---|---|---|---|
| **permeability** | **Vascular volume** | **VP/VV** | **Fold change** | |
| **A-5RT3CTRL** | 0.76 ± 0.09* | 0.82 ± 0.17** | 0.927 | 5.332 |
| **A-5RT3ΔANGPTL4** | 0.073 ± 0.012 | 0.42 ± 0.09 | 0.174 | 1.000 |

| | | | | |
|---|---|---|---|---|
| Data represent the mean±SD of 15 animals. *, *P* < 0.001; **, *P* < 0.01 | | | | |

Confluent primary human microvascular ECs were cultured in 3-day-old CM from either A-5RT3CTRL or A-5RT3ΔANGPTL4 cells. EC junction integrity was disrupted when the cells were cultured in A-5RT3CTRL CM, but not in A-5RT3ΔANGPTL4 CM, as indicated by ZO-1 staining. Junction integrity was maintained in the presence of anticANGPTL4 monoclonal antibody, suggesting that tumor-secreted cANGPTL4 plays a major role in endothelial junction disruption (Figure 1I). We found that EC-EC junction integrity was disrupted when the cells were treated with only rh-cANGPTL4 (6 µg/ml) in the presence of either cycloheximide or actinomycin D, indicating that *de novo* protein synthesis and transcription were not required (Figure S2C). Treatment with cycloheximide, actinomycin D or vehicle alone did not induce any vascular lesions (Figure S2C). This disruption was mirrored by a decreased in the transendothelial electrical resistance (TER) of the monolayer, suggesting a rapid and dose-dependent increase in paracellular permeability (Figure 1J). No change in TER was observed in the PBS control (Figure 1J). This effect was not due to apoptosis, even after 6 h of rhcANGPTL4 treatment (Figure S2D). Altogether, these results indicate that tumorsecreted cANGPTL4 disrupts endothelial integrity.

### cANGPTL4 interacts with integrin α5β1, VE-cadherin and claudin-5.

VE-cadherin and claudin-5 contribute to the control of paracellular permeability, while integrin α5β1, which is localized to EC-EC junctions, also mediates endothelial leakiness.4,6 Hence, we hypothesized that tumor-derived cANGPTL4 induces vascular leakiness and ultimately disrupts the endothelium barrier via direct interactions with specific junction proteins.
Surface plasmon resonance (SPR) analysis revealed that cANGPTL4 interacted with VEcadherin (KD=1.12x10-7 M), claudin-5 (KD=5.87x10-8 M) and integrin α5β1 (KD= 1.26x10-8 M), but not with occludin or JAM-A (Figure 2A). More significantly, cANGPTL4 targeted the VE-cadherin EC1 domain and claudin-5 ECD1 domain, which are responsible for homophilic interactions between adjacent ECs, suggesting that the interactions of cANGPTL4 with VE-cadherin and claudin-5 could play an important role in regulating vascular permeability (Figure 2B).21,22 No interactions were observed between cANGPTL4 and integrin β316,17 or other domains of VE-cadherin (EC3) and claudin-5 (ECD2), which also served as controls (Figure 2B). The observations of these interactions were corroborated by blocking with neutralizing antibodies against integrin α5β1 (Figure 2C), VE-cadherin (Figure 2D), claudin-5 (Figures 2E) and cANGPTL4 (Figures S3A-C). An *in situ* proximity ligation assay (PLA) detected complexes of integrin α5β1, claudin-5 or VE-cadherin with cANGPTL4 in both EC (integrin 22.50±5.01; VE-cadherin 16.43±2.46; claudin-5 12.35±3.44 PLA spots/cell calculated from 250 cells) and A-5RT3CTRL-induced tumor biopsies (Figure 2F, S3D), confirming that these interactions also exists *in vivo.* No PLA signals, i.e. no interactions were observed between CD31 and cANGPTL4, claudin-5, VE-cadherin or integrin α5β1 (Figure S3E). cANGPTL4 interacted directly with the EC1 and ECD1 adhesive domains of VEcadherin and claudin-5, respectively, which may lead to disruption of intercellular VEcadherin and claudin-5 cluster formation.23 To examine this, membrane fractions of confluent ECs treated with cANGPTL4 were extracted, resolved under native conditions, and probed with either anti-VE-cadherin or anti-claudin-5. cANGPTL4 treatment resulted in smaller protein bands when compared with PBS (Figure 2G). Treatment with EDTA, which disrupts intercellular adhesion, served as a positive control for declustered VEcadherin and claudin-5. These findings confirm that cANGPTL4 interacts with integrin α5β1, VE-cadherin and claudin-5 to disrupt intercellular adhesion.

### cANGPTL4 modulates endothelial integrity in a temporally sequential mechanism.

Next, we sought a mechanistic explanation for the effect of cANGPTL4 on the endothelium. We monitored the temporal interactions of cANGPTL4 and its three binding partners through kinetic PLA. A bimodal interaction profile of the cANGPLT4:integrin α5β1 complex was detected within 30-60 min, followed by cANGPTL4:VE-cadherin and cANGPTL4:claudin-5 at 120 min post-cANGPTL4 treatment (Figure 3A). *In vitro* co-immunoprecipitation of cANGPTL4 and its various interacting partners validated our PLA and SPR results (Figure 3B). Except for integrin α5β1, which showed elevated expression only 2 h post-cANGPTL4 treatment, no change in protein level was observed for any other cANGPTL4-interacting partner, indicating that the bimodal cANGPTL4-protein interaction profiles did not result from increased protein expression (Figure S4A). We also showed that the initial cANGPTL4:integrin α5β1 interactions resulted in the complex being internalized into the cytoplasm at 40 min, followed by the internalization of the cANGPTL4:VE-cadherin and cANGPTL4:claudin- 5 complexes between 120-150 min to result in the abrogation of the endothelial junctions (Figure 3C). This bimodal effect was not observed with the control treatment (Figure S4B). Notably, cANGPTL4:integrin α5β1 formation at 30-60 min coincided with a TER decrease, suggesting increased permeability (Figure 1K). Integrin β3, which does not interact with cANGPTL4, was internalized after 2 h in both vehicle and cANGPTL4- treated cells. Similar observations were also reported in wild-type and ANGPTL4- knockdown keratinocytes17, suggesting that this may be inherent to either integrin β3 or the experimental procedure. Thus, our results suggest that the initial interaction of cANGPTL4 with integrin α5β1 may be required to facilitate the subsequent interactions to mediate vascular disruption.

### Inhibition of the cANGPTL4:integrin α5β1 complex delays cANGPTL4 interactions with VE-cadherin and claudin-5.

Antibody to integrin α5β1 increased endothelial leakiness, but was insufficient to induce microscopic lesions at endothelial junctions. Thus, we sought to understand the impact of the upstream cANGPTL4:integrin α5β1 formation on the disruption of endothelial integrity. In the presence of monoclonal anti-integrin α5β1, cANGPTL4 required twice the length of time (6 h) to induce lesions along the EC-EC contact compared to the cANGPTL4 alone (Figure 4A). Anti-integrin α5β1 blocked the interaction with cANGPTL4 (Figure 2E). ECs treated with anti-integrin α5β1 alone did not induce any lesions, even after 6 h (Figure 4A). These results were corroborated by our TER data, which showed that the addition of anti-integrin α5β1 and cANGPTL4 (6 µg/ml) to ECs resulted in reduced endothelial permeability when compared with ECs treated only with 6 µg/ml cANGPTL4 (Figure 4B). Accordingly, anti-integrin α5β1 caused a concomitant delay of ∼90 min in the interaction profile of cANGPTL4:VE-cadherin and cANGPTL4:claudin-5 when compared to control (compare Figures 4C and 3A). The mean fold change (0.4±0.12) in the interactions between cANGPTL4 and these 2 proteins remained similar, albeit with a non-significant decrease of ∼10% for VE-cadherin, suggesting that interactions with these 2 proteins are required for endothelial disruption (Figure 4C). Thus, we treated HMVECs with cANGPTL4 for 90 min, which allowed for cANGPTL4:integrin α5β1 interactions and initiated endothelial leakiness, followed by its removal. Immunostaining for ZO-1 revealed that cANGPTL4:integrin α5β1 interactions alone is not sufficient to induce endothelial disruption, and that the later interactions are necessary for the observed disruption. (Figure 4D). Interestingly, we observed from our TER data that the removal of rhcANGPTL4 promoted a "reversal effect" on endothelial permeability, conceivably due to the "re-zipping" of the EC-EC contacts, thus reinforcing the important of cANGPTL4:integrin α5β1 complex formation in the entire process of endothelial disruption (Figure 4E). Hence, our results suggest that although integrin α5β1 is an essential player, its interaction with cANGPTL4 alone is insufficient to induce an endothelial disruption/lesion. The data suggest that the binding of cANGPTL4 to integrin α5β1 triggers internalization of the complex and "un-zipping" of the endothelial junctions to first promote endothelial leakiness. This facilitates the subsequent cANGPTL4 interactions with the adhesive domains of VE-cadherin and claudin-5 that weaken their homophilic contacts, resulting in their declustering and internalization and finally culminating in the disruption of EC-EC contacts.

### cANGPTL4-induced disruption of the endothelium involves integrin-mediated PAK/Rac signaling.

The pivotal role of integrin α5β1 in the process of EC-EC contact disruption lead us to examine the detailed molecular events resulting from the cANGPTL4:intergrin α5β1 complex formation. *In vitro* co-immunoprecipitation of cANGPTL4 with activated integrin β1 (antibody clone Huts-21) indicated that integrin was activated upon association with cANGPTL4 (Figure 5A, B). No interactions were observed between cANGPTL4 and Tie1, Tie2, integrin β3 or the junction protein JAM-C (Figure 5B). Rac-GTP and PAK are downstream mediators of integrin signaling, whose roles in endothelial contractility and barrier function are well recognized.24,25 Our coimmunoprecipitation results showed that ANGPTL4-activated integrin β1 also stimulates the activation of Rac1 and phosphorylation of PAK, when compared to PBS-treated ECs (Figure 5C). The increased cANGPTL4:integrin α5β1 interaction and activation of PAK/Rac1 signaling coincided with endothelial leakiness (Figure 1J), possibly by modulating integrin-mediated intercellular adhesion and contractility(24,26).

To further underscore the role of Rac1, we transfected ECs with either constitutive active Rac1 (G12V) or dominant-negative Rac1 (T17N). Transfection of ECs with Rac1 (G12V) accelerated the formation of cANGPTL4:VE-cadherin and cANGPTL4:claudin-5 complexes, while the expression of Rac1 (T17N) delayed the complex delayed the complex formation (compare Figures 3A and 5D). This suggests that cANGPTL4 regulates endothelial integrity via the integrin α5β1 □mediated Rac/PAK signaling axis, which functions via signaling cross-talk to facilitate cANGPTL4 interactions with VEcadherin and claudin-5, ultimately leading to vascular disruption.

### cANGPTL4 modulates the nuclear translocation of β-catenin.

The perturbations and disassembly of VE-cadherin result in the translocation of membrane β-catenin to the nucleus.27,28 Hence, we asked if cANGPTL4-induced declustering of VE-cadherin and claudin-5 resulted in nuclear translocation of β-catenin. In PBS-treated ECs, β-catenin formed a continuous lining along the cell margin as indicated by the immunofluorescence staining and intensity plot (Figure 6A and S5A), whereas treatment with either cANGPTL4 (6 µg/ml for 3 h) or 2.5 mM EDTA (positive control) resulted in the disappearance of the β-catenin margin and accumulation in the nuclei, indicating cell-cell junction disruption (Figure 6A and S5A). To better define the localization of β-catenin, we performed PLA that showed β-catenin in the nuclei at 180 min (β-catenin spots/nucleus increases from 1.2±0.81 at 0 min to 17.5±2.68 at 180 min, while those outside the nucleus decreases from 26.3±3.29 to 9.44±3.98); this timing coincided with the declustering of VE-cadherin and claudin-5 (Figure 6B). Immunoblot analysis corroborated our PLA data (Figure 6C). Taken together, our data indicate that tumor-derived cANGPTL4 is a permeability-inducing factor capable of triggering endothelial disruption via sequential interactions with integrin α5β1, VE-cadherin and claudin-5, resulting in the accumulation of nuclear β-catenin (Figure 6D).

### cANGPTL4 promotes metastasis to the lungs in vivo.

To further investigate the role of cANGPTL4 in the metastatic process and to substantiate its biological relevance in cancer, we examined its effect on lung metastasis using two different mice models. Wildtype C57BL/6J mice (n=12 per group) were injected intravenously with either B16F10CTRL or B16F10ΔANGPTL4 cells (2 x 106) and treated intravenously with either PBS or rh-cANGPTL4. Mice inoculated with B16F10ΔANGPTL4 had significantly fewer lung metastases compared with B16F10CTRL-injected mice. Intravenous administration of rhcANGPTL4 enhanced lung metastases in both cases, as evidenced by real-time PCR analysis for melanin A, a marker of melanoma (Figure 7A, B). Cross-section staining of the lung tissues corroborated our *in vivo* data (Figure 7C and S5B), underscoring the biological metastatic potential of cANGPTL4 in tumor cells (Figure 7A, B). Additionally, we examined lung metastases using wild-type (*ANGPTL*+/+) and *ANGPTL4*-knockout (*ANGPTL4-*/*-*) mice injected with either 0.5 x 105
B16F10CTRL or B16F10ΔANGPTL4. There was a significantly reduced number of tumor nodules in the lungs of *ANGPTL4-*/*-* when compared with *ANGPTL4*+/+ mice, suggesting that metastasis of B16F10CTRL was impaired (Figure 7D, E). Altogether, our data indicate cANGPTL4 is a pro-metastatic tumor-derived factor and provide new insights into the mechanistic action of this protein in cancer.

Metastasis requires dynamic tumor-endothelium communication through tumor secreted vascular permeability factors to breach the endothelial barrier, thereby allowing the migration and invasiveness of the tumor cells across the endothelium. Hence, a better understanding of the mechanism by which such vascular permeability factors disrupt endothelial integrity could offer new therapeutic strategies. The mechanism by which ANGPTL4 modulates endothelial junction integrity remains unknown. Here, we provide insight into the heterotypic role of cANGPTL4 in vascular integrity. We show that cANGPTL4 disrupts intercellular adhesion via a novel integrin α5β1-mediated Rac/PAK signaling pathway and the declustering and internalization of VE-cadherin and claudin-5, resulting in nuclear accumulation of β-catenin. Using two different mouse models, our *in vivo* vascular permeability and metastatic assays confirmed that cANGPTL4 acts as a vascular permeability factor by inducing vascular leakiness and disruption. Interestingly, ANGPTL4-deficient tumors also showed decreased vascularisation, suggesting that cANGPTL4 may facilitate metastasis through increased vascular permeability and volume.

EC-EC adhesion is mediated mainly by the α5β1 integrin and EC-specific adhesion complexes containing VE-cadherin and claudin-5. 6,29 Our work shows that cANGPTL4 interacts with these three adhesive complexes in a sequential manner to disrupt the endothelial junctions. A major route by which integrins regulate cell-cell contacts is the Rac/PAK pathway, leading to modulation of EC contractility.24,30 31 We show that the initial interaction between cANGPTL4 and integrin α5β1 activates Rac and phosphorylate PAK, weakening cell-cell contacts and increasing endothelial leakiness. This facilitates cANGPTL4 access to the adhesive domains of VE-cadherin and claudin-5 to induce declustering of the adhesion molecules resulting in vascular disruption. We and others have shown that ANGPTL4 does not bind the angiopoietin receptor Tie1 or Tie2 to antagonize its action. Angiopoietin-1-mediated activation of Rac reduces vascular leak in the context of VEGF-induced vascular permeability. Thus, the role of Rac in vascular permeability is complex. The ligand and the cellular context that activate Rac will dictate its effect on vascular junction integrity.

The activation of Rac/PAK signaling trigger by cANGPTL4-bound integrin β1 is necessary to facilitate cANGPTL4 access to VE-cadherin and claudin-5 and to trigger vascular disruption. By preventing the association of cANGPTL4 with integrin β1 by anti-integrin β1, we observed delayed cANGPTL4:VE-cadherin and cANGPTL4:claudin- 5 complexes formation and a concomitant decreased vascular permeability. Similar delay in cANGPTL4:VE-cadherin and cANGPTL4:claudin-5 complexes formation were also detected in ECs expressing dominant-negative Rac1, without affecting the cANGPTL4:integrin β1 formation. This indicates that the Rac/PAK signaling acts downstream of integrin and facilitates cANGPTL4 interactions with VE-cadherin and claudin-5. Interestingly, by preventing the subsequent cANGPTL4 interaction with VEcadherin and claudin-5, the initial increased in vascular permeability due to the activation of integrin-mediated Rac/PAK signaling did not propagated to observable vascular lesions. This confirms that Rac/PAK signaling is necessary to potentiate the vascular disruptive effect of cANGPTL4.

The interactions of cANGPTL4 with the VE-cadherin ED1 and claudin-5 EDC1 domains disrupt VE-cadherin and claudin-5 clustering. The EC intercellular clustering of VE-cadherin and claudin-5, mediated by their extracellular domains, is important for the maintenance of a semi-permeable boundary between the bloodstream and the tumor (21, 22, 32). Our results also reveal a time-dependent cellular trafficking of β-catenin from the membrane to the nucleus in cANGPTL4-treated ECs, coinciding with endothelial disruption. The interactions between the cytoplasmic tail of VE-cadherin and the catenins are essential for the proper functioning of stable AJs (28). The nuclear accumulation of β-catenin in leaky endothelium and release of β-catenin from AJ junctions into the nucleus is consistent with the declustering of AJs (27,28,33). Moreover, the membrane-liberated β-catenin induces gene transcription that contributes to angiogenic responses of ECs following episodes of vascular leakage that promotes tumor metastasis (34-36).

The role of ANGPTL4 in vascular leakiness, and thus metastasis, is controversial, and efforts to confirm it have been further hampered by the lack of understanding of the mechanistic action of ANGPTL4. We show that cANGPTL4 behaves as a vascular permeability factor and disrupts endothelial integrity via integrin α5β1-mediated Rac/PAK signaling and the declustering and internalization of VEcadherin and claudin-5. The reason for the apparent discrepancy is unclear, but it may be due to the position of fusion tags in recombinant ANGPTL4. Studies that suggest an inhibitory role of ANGPTL4 in vascular leakiness have used a C-terminal fusion ANGPTL4, while our work and that of others who have presented evidence that ANGPTL4 is a pro-metastatic agent that induces endothelial permeability has utilize either an N-terminal fusion ANGPTL4 or un-tagged ANGPTL4. A dysfunctional endothelium usually presents as a leaky or disrupted vasculature and is characteristic of many pathological conditions, including inflammatory edema, diabetes, atherosclerosis, and cancer metastasis (33,38) The disruption of EC-EC contacts is a major route exploited by primary tumors to promote metastasis. Our findings suggest that targeting cANGPTL4 to treat metastasis and vascular-related diseases could be a viable option.

Those skilled in the art will appreciate that the invention described herein is susceptible to variations and modifications other than those specifically described. The invention includes all such variation and modifications. The invention also includes all of the steps, features, formulations and compounds referred to or indicated in the specification, individually or collectively and any and all combinations or any two or more of the steps or features.
Each document, reference, patent application or patent cited in this text is expressly incorporated herein in their entirety by reference, which means that it should be read and considered by the reader as part of this text. That the document, reference, patent application or patent cited in this text is not repeated in this text is merely for reasons of conciseness.
Any manufacturer's instructions, descriptions, product specifications, and product sheets for any products mentioned herein or in any document incorporated by reference herein, are hereby incorporated herein by reference, and may be employed in the practice of the invention.
The present invention is not to be limited in scope by any of the specific embodiments described herein. These embodiments are intended for the purpose of exemplification only. Functionally equivalent products, formulations and methods are clearly within the scope of the invention as described herein.
The invention described herein may include one or more range of values (e.g. size, concentration etc). A range of values will be understood to include all values within the range, including the values defining the range, and values adjacent to the range which lead to the same or substantially the same outcome as the values immediately adjacent to that value which defines the boundary to the range. Throughout this specification, unless the context requires otherwise, the word "comprise" or variations such as "comprises" or "comprising", will be understood to imply the inclusion of a stated integer or group of integers but not the exclusion of any other integer or group of integers. It is also noted that in this disclosure and particularly in the claims and/or paragraphs, terms such as "comprises", "comprised", "comprising" and the like can have the meaning attributed to it in U.S. Patent law; e.g., they can mean "includes", "included", "including", and the like; and that terms such as "consisting essentially of" and "consists essentially of" have the meaning ascribed to them in U.S. Patent law, e.g., they allow for elements not explicitly recited, but exclude elements that are found in the prior art or that affect a basic or novel characteristic of the invention.
Other definitions for selected terms used herein may be found within the detailed description of the invention and apply throughout. Unless otherwise defined, all other scientific and technical terms used herein have the same meaning as commonly understood to one of ordinary skill in the art to which the invention belongs.

### References

1. Yilmaz M, Christofori G, Lehembre F. Distinct mechanisms of tumor invasion and metastasis. Trends Mol Med 2007;13(12):535-541.
2. Dejana E. Endothelial cell-cell junctions: happy together. Nat Rev Mol Cell Biol 2004;5(4):261-270.
3. Komarova Y, Malik AB. Regulation of endothelial permeability via paracellular and transcellular transport pathways. Annu Rev Physiol 2010;72:463-493.
4. Bazzoni G, Dejana E. Endothelial cell-to-cell junctions: molecular organization and role in vascular homeostasis. Physiol Rev 2004;84(3):869-901.
5. Desgrosellier JS, Cheresh DA. Integrins in cancer: biological implications and therapeutic opportunities. Nat Rev Cancer 2010;10(1):9-22.
6. Lampugnani MG, Resnati M, Dejana E, Marchisio PC. The role of integrins in the maintenance of endothelial monolayer integrity. J Cell Biol 1991 ;112(3):479-490.
7. Joyce JA, Pollard JW. Microenvironmental regulation of metastasis. Nat Rev Cancer 2009;9(4):239-252.
8. Guo W, Giancotti FG. Integrin signalling during tumour progression. Nat Rev Mol Cell Biol 2004;5(10):816-826.
9. Thijssen VL, Barkan B, Shoji H et al. Tumor cells secrete galectin-1 to enhance endothelial cell activity. Cancer Res 2010;70(15):6216-6224.
10. Padua D, Zhang XHF, Wang Q et al. TGFbeta primes breast tumors for lung metastasis seeding through angiopoietin-like 4. Cell 2008;133(1):66-77.
11. Galaup A, Cazes A, Le JS et al. Angiopoietin-like 4 prevents metastasis through inhibition of vascular permeability and tumor cell motility and invasiveness. Proc.Natl.Acad.Sci.U.S.A 2006;103(49):18721-18726.
12. Ito Y, Oike Y, Yasunaga K et al. Inhibition of angiogenesis and vascular leakiness by angiopoietin-related protein 4. Cancer Res. 2003;63(20):6651-6657.
13. Hu Z, Fan C, Livasy C et al. A compact VEGF signature associated with distant metastases and poor outcomes. BMC Med 2009;7:9.
14. Minn AJ, Gupta GP, Siegel PM et al. Genes that mediate breast cancer metastasis to lung. Nature 2005;436(7050):518-524.
15. Minn AJ, Gupta GP, Padua D et al. Lung metastasis genes couple breast tumor size and metastatic spread. Proc Natl Acad Sci U S A 2007;104(16):6740-6745.
16. Zhu P, Tan MJ, Huang RL et al. Angiopoietin-like 4 protein elevates the prosurvival intracellular O2(-):H2O2 ratio and confers anoikis resistance to tumors. Cancer Cell 2011 ;19(3):401-415.
17. Goh YY, Pal M, Chong HC et al. Angiopoietin-Like 4 Interacts with Integrins {beta}1 and {beta}5 to Modulate Keratinocyte Migration. Am J Pathol 2010;177(6):2791-2803.
18. Goh YY, Pal M, Chong HC et al. Angiopoietin-like 4 interacts with matrix proteins to modulate wound healing. J Biol Chem 2010;285(43):32999-33009.
19. Ito Y, Oike Y, Yasunaga K et al. Inhibition of angiogenesis and vascular leakiness by angiopoietin-related protein 4. Cancer Res. 2003;63(20):6651-6657.
20. Allalou A, Wählby C. BlobFinder, a tool for fluorescence microscopy image cytometry. Comput Methods Programs Biomed 2009;94(1):58-65.
21. Wen H, Watry DD, Marcondes MCG, Fox HS. Selective decrease in paracellular conductance of tight junctions: role of the first extracellular domain of claudin-5. Mol Cell Biol 2004;24(19):8408-8417.
22. Taveau JC, Dubois M, Le Bihan O et al. Structure of artificial and natural VEcadherin- based adherens junctions. Biochem Soc Trans 2008;36(Pt 2):189-193.
23. Taddei A, Giampietro C, Conti A et al. Endothelial adherens junctions control tight junctions by VE-cadherin-mediated upregulation of claudin-5. Nat Cell Biol 2008;10(8):923-934.
24. Stockton RA, Schaefer E, Schwartz MA. p21-activated kinase regulates endothelial permeability through modulation of contractility. J Biol Chem 2004;279(45):46621- 46630.
25. Galan Moya EM, Le Guelte A, Gavard J. PAKing up to the endothelium. Cell Signal 2009;21 (12):1727-1737.
26. Gimond C, van Der Flier A, van Delft S et al. Induction of cell scattering by expression of beta1 integrins in beta1-deficient epithelial cells requires activation of members of the rho family of GTPases and downregulation of cadherin and catenin function. J Cell Biol 1999;147(6):1325-1340.
27. Beckers CML, Garcia-Vallejo JJ, van Hinsbergh VWM, van Nieuw Amerongen GP. Nuclear targeting of beta-catenin and p120ctn during thrombin-induced endothelial barrier dysfunction. Cardiovasc Res 2008;79(4):679-688.
28. Conacci-Sorrell M, Zhurinsky J, Ben-Ze'ev A. The cadherin-catenin adhesion system in signaling and cancer. J Clin Invest 2002;109(8):987-991.
29. Hanahan D, Weinberg RA. The hallmarks of cancer. Cell 2000;100(1):57-70.
30. Miyamoto Y, Reddig P, Juliano RL. Regulation of signal transduction by integrins. Handb Exp Pharmaco/ 2004;(165):197-216.
31. Hoang MV, Nagy JA, Senger DR. Active Rac1 improves pathologic VEGF neovessel architecture and reduces vascular leak: mechanistic similarities with angiopoietin-1. Blood 2011 ;117(5):1751-1760.
32. Sumpio BE, Riley JT, Dardik A. Cells in focus: endothelial cell. Int J Biochem Cell Biol 2002;34(12):1508-1512.
33. Harris ES, Nelson WJ. VE-cadherin: at the front, center, and sides of endothelial cell organization and function. Curr Opin Cell Biol 2010;22(5):651-658.
34. Skurk C, Maatz H, Rocnik E, Bialik A, Force T, Walsh K. Glycogen-Synthase Kinase3beta/beta-catenin axis promotes angiogenesis through activation of vascular endothelial growth factor signaling in endothelial cells. Circ Res 2005;96(3):308-318.
35. Conacci-Sorrell M, Zhurinsky J, Ben-Ze'ev A. The cadherin-catenin adhesion system in signaling and cancer. J Clin Invest 2002;109(8):987-991.
36. Beckers CML, Garcia-Vallejo JJ, van Hinsbergh VWM, van Nieuw Amerongen GP. Nuclear targeting of beta-catenin and p120ctn during thrombin-induced endothelial barrier dysfunction. Cardiovasc Res 2008;79(4):679-688.
37. Le Jan S, Amy C, Cazes A et al. Angiopoietin-like 4 is a proangiogenic factor produced during ischemia and in conventional renal cell carcinoma. Am J Pathol 2003;162(5):1521-1528.
38. Fukumura D, Duda DG, Munn LL, Jain RK. Tumor microvasculature and microenvironment: novel insights through intravital imaging in pre-clinical models. Microcirculation 2010;17(3):206-225.

## Claims

1. A method of modulating endothelial cell permeability by controlling the concentration or activity of Angiopoietin-like 4 (ANGPTL4) in the cell environment.

2. The method as claimed in claim1 wherein the cell environment is *in vivo.*

3. The method as claimed in claim 1 or 2 wherein the cell environment is *in vitro.*

4. The method as claimed in any one of claims 1 to 3 wherein increasing the concentration or activity of Angiopoietin-like 4 (ANGPTL4) and/or cANGPTL4 induce endothelial cell permeability.

5. The method as claimed in any one of claims 1 to 3 wherein removing, degrading or neutralising the concentration or activity of Angiopoietin-like 4 (ANGPTL4) and/or cANGPTL4 inhibits endothelial cell permeability.

6. The method of claim 5 wherein Angiopoietin-like 4 (ANGPTL4) and/or cANGPTL4 is removed, degraded or neutralised by a cANGPT4L specific antibody.

7. The method of claim 6 wherein the antibody is catalytic.

8. A method for treating a patient to at least reduce endothelial cell permeability, which comprises the step of:
a. contacting the cell with an antagonist to cANGPTL4.

9. The method of claim 8 wherein the antagonist is an antibody to cANGPTL4.

10. The method of claim 9 wherein the antibody is a neutralizing antibody to cANGPTL4.

11. The method of any one of claims 8 to 10 wherein the antagonist interferes with increased endothelial cell permeability.

12. The method of claim 8 wherein the antagonist comprises a compound that engages an cANGPTL4 domain of ANGPTL4 preventing increased endothelial cell permeability.

13. A method comprising the steps of:
a. preparing an antibody using an adjuvant and a cANGPTL4 polypeptide; and
b. administering the antibody to a patient suffering from vascular-related diseases.

14. The method of claim 13 wherein the vascular-related diseases is selected from the group consisting of inflammatory edema, diabetes, and atherosclerosis.

15. The method of claim 13 wherein the vascular-related diseases is cancer metastasis.

16. The method of claim 15 further comprising administering an anticancer agent to the patient.

17. A composition comprising a therapeutically effective amount of a modulator of cANGPTL4 concentration.

18. The composition of claim 17 wherein the modulator is an antagonist to cANGPTL4.

19. The composition of claim 18 wherein the antagonist is an antibody to cANGPTL4.

20. The composition of claim 19 wherein the antibody is a catalytic antibody to cANGPTL4.

21. The composition of claim 17 wherein the modulator comprises a compound that engages a cANGPTL4 domain of ANGPTL4 preventing increased endothelial cell permeability.

22. The composition of any one of claims 17 to 21 for use as a medicament for treating a patient suffering from vascular-related diseases.

23. A use of the composition claimed in any one of claims 18 to 24 in the manufacture of a medicament for treating a patient suffering from vascular-related diseases.

24. The use of claim 23 wherein the vascular-related diseases is selected from the group consisting of inflammatory edema, diabetes, and atherosclerosis,.

25. The use of claim 23 wherein the vascular-related diseases is cancer metastasis.

26. The use of claim 25 further comprising the step of administering an anticancer agent to the patient.

27. A method of determining the extent of endothelial cell permeability in a sample comprising the step of:
a. measuring the amount of ANGPTL4 protein is in the sample.

28. The method of claim 27 further comprising the step of:
b. determining the patient's treatment according to whether the amount of ANGPTL4 protein is indicative of increased endothelial cell permeability.
